(19) Europäisches Patentamt
European Patent Office
Office européen des brevets

(11) **EP 4 485 300 A1**

(12) **EUROPEAN PATENT APPLICATION**
published in accordance with Art. 153(4) EPC

(43) Date of publication:
**01.01.2025 Bulletin 2025/01**

(21) Application number: **23759959.2**

(22) Date of filing: **21.02.2023**

(51) International Patent Classification (IPC):
**G06Q 10/04** (2023.01)      **G06Q 50/10** (2012.01)
**G16H 10/00** (2018.01)

(52) Cooperative Patent Classification (CPC):
**G06Q 10/04; G06Q 50/10; G16H 10/00**

(86) International application number:
**PCT/JP2023/006145**

(87) International publication number:
**WO 2023/162958 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.02.2022 JP 2022028697**

(71) Applicant: **Kao Corporation
Tokyo 103-8210 (JP)**

(72) Inventors:
• **MARUYAMA, Hiroshi
Tokyo 100-0004 (JP)**
• **GAO, Zhengyan
Tokyo 100-0004 (JP)**

• **HAYASHI, Kohei
Tokyo 100-0004 (JP)**
• **MAEDA, Shinichi
Tokyo 100-0004 (JP)**
• **KAWAKAMI, Aya
Tokyo 103-8210 (JP)**
• **BITO, Kotatsu
Tokyo 103-8210 (JP)**
• **HIBI, Masanobu
Tokyo 131-8501 (JP)**
• **KATADA, Shun
Tokyo 131-8501 (JP)**

(74) Representative: **Hoffmann Eitle
Patent- und Rechtsanwälte PartmbB
Arabellastraße 30
81925 München (DE)**

(54) **INFORMATION PROCESSING DEVICE, METHOD, AND PROGRAM**

(57)     A missing value of body information is estimated. An information processing apparatus according to one embodiment of the present disclosure comprises at least one memory and at least one processor. The at least one processor executes acquiring an estimated value of a first attribute by inputting body information in which a value of the first attribute is missing to at least one trained model, and acquiring an estimated value of a second attribute by inputting body information in which a value of the second attribute is missing to the at least one trained model, the second attribute being different from the first attribute. The first attribute and the second attribute are body information other than basic information.

FIG.2

EP 4 485 300 A1

## Description

Field of the Invention

The present disclosure relates to an information processing apparatus, a method, and a program.

Background of the Invention

Conventionally, body information such as check results in a medical checkup has been utilized. Specifically, a service using values of a plurality of attributes of body information (e.g., a plurality of check results) has been performed. However, all subjects do not always measure values of all attributes of body information, and values of some attributes of the body information are missing in some cases. For example, there are cases where subjects are not subjected to optional checks in a medical checkup, and items of the medical checkup to be handled are different between facilities.

Citation List

Patent Literature

Patent Literature 1: Japanese Patent Application Laid-open No. 2012-064087

Non-Patent Literature

Non-Patent Literature 1: Handling Incomplete Heterogeneous Data using VAEs, [online], Internet <URL: https://arxiv.org/pdf/1807.03653.pdf>

Summary of the Invention

Technical Problem

An object of the present disclosure is to estimate a missing value of body information.

Solution to Problem

An information processing apparatus according to one embodiment of the present disclosure comprises at least one memory and at least one processor. The at least one processor executes acquiring an estimated value of a first attribute by inputting body information in which a value of the first attribute is missing to at least one trained model, and acquiring an estimated value of a second attribute by inputting body information in which a value of the second attribute is missing to the at least one trained model, the second attribute being different from the first attribute. The first attribute and the second attribute are body information other than basic information.

Brief Description of the Drawings

[Fig. 1] Fig. 1 is an example of the overall configuration according to one embodiment of the present disclosure.
[Fig. 2] Fig. 2 is a functional block diagram of an information processing apparatus according to one embodiment of the present disclosure.
[Fig. 3] Fig. 3 is a diagram for describing training data according to one embodiment of the present disclosure.
[Fig. 4] Fig. 4 is a diagram for describing training data according to one embodiment of the present disclosure.
[Fig. 5] Fig. 5 is a diagram for describing body information including a missing value and body information including an estimated value according to one embodiment of the present disclosure.
[Fig. 6] Fig. 6 is a diagram showing an estimation example according to one embodiment of the present disclosure.
[Fig. 7] Fig. 7 is a flowchart of training processing according to one embodiment of the present disclosure.
[Fig. 8] Fig. 8 is a flowchart of estimation processing according to one embodiment of the present disclosure.
[Fig. 9] Fig. 9 is a hardware configuration diagram of the information processing apparatus according to one embodiment of the present disclosure.

Detailed Description of the Invention

Hereinafter, an embodiment of the present disclosure will be described with reference to the drawings.

<Explanation of Terms>

· In this specification, "body information" refers to information regarding the mind and body of a human and includes information that can be an index of the body information. For example, the body information is a check result of a medical checkup, receipt information (e.g., information regarding a medical action performed by a medical institution on a patient, a name of an injury or disease, and medication), information collected as a response to a questionnaire on the body, and the like. For example, the "body information" can include "basic information", "information regarding a physical condition", and "information regarding a psychological state". Note that, for at least the "basic information" and the "information regarding a physical condition", the present disclosure can be applied to body information of animals other than humans.
· In this specification, the "basic information" is basic information regarding a body that can be recognized by a person himself/herself. In this embodiment, the "basic information" preferably includes sex, age, height, and weight.
· In this specification, the "information regarding a physical condition" is information indicating a physical condition of a human body. For example, the "information regarding a physical condition" is: information regarding general blood, liver function, lipid, metabolic system, blood pressure, and the like, which are check items of a medical checkup; and scores, numerical values, and classification information that are obtained from all or part of the body by measurement, and scores, numerical values, and classification information that are obtained from medical interviews or inquiry, which do not include the basic information.

For example, the "information regarding a physical condition" is information regarding body frame/body composition values (body fat, muscle mass, visceral fat mass, and the like), blood test values (neutral fat, total cholesterol, HDL cholesterol, LDL cholesterol, blood glucose, HbA1c, ALT, red blood cell count, aspartate aminotransferase, alanine aminotransferase, γ-glutamyltranspeptidase, and alkaline phosphatase), various hormones (cortisol, triiodothyronine, thyroxine, dehydroepiandrosterone, testosterone, estradiol, progesterone, follicle stimulating hormone, luteinizing hormone, prolactin, and the like), vascular function (index of arteriosclerosis, stenosis/occlusion index of artery of lower limb), glucose metabolism function (blood glucose, insulin, glucagon, GLP-1, and GIP), cognitive function (function test (Cognitrax (registered trademark)), and blood D- or L-amino acids), motor function (grip strength, total motor performance, walking function (speed, stride length, and pitch), etc.), immune-related indices (lymphoid subset analysis, NK cellular activity, cytokine analysis, etc.), disease and illness information (presence or absence of disease and illness, details of physical examination, medication, etc.), amount of activity (calorie consumption per day, and number of steps per day), sleep-related indices (scores by questionnaire), productivity (scores by questionnaire), diet/nutritional status (calories burned, PFC balance, etc.), menstrual-related indices (stage determination, and disability-level determination by medical interview and inquiry), menopausal disorder-related indices (stage determination, and disability-level determination by inquiry), hair (diameter, and degree of waviness), skin condition (blood flow velocity of skin, degree of glycation of skin, and ceramide content), and body odor (quantification of odor components by gas chromatography), oriental medical constitution classification (clustering by questionnaire), microbiota information (bacterial quantity and composition ratio of oral cavity, intestine, scalp, and skin), the degree of progress of thin hair and alopecia (score by photographic determination), DNA and RNA information, overactive bladder/urinary incontinences (score by questionnaire), and the like.

· In this specification, the "information regarding a psychological state" is information indicating a psychological state of a human, and are a determination by a medical interview, a questionnaire, a test, or a marker, a score, a numerical value, and information that can be classified. For example, the "information regarding a psychological state" is information regarding personality traits (Big Five personality traits: openness to experience, conscientiousness, extroversion, agreeableness, and neuroticism), stress (stress response scores (stress level) etc. in the Brief Job Stress Questionnaire), mental fatigue, mental health (manic-depressive illness), the level of happiness (scores according to QOL questionnaire), and the like.

In this specification, each attribute included in the "information regarding a physical condition", the "information regarding a psychological state", and the "basic information" is expressed by a score, a numerical value, and a classification, and it is known that there are relationships between the attributes. For example, it is estimated that the "amount of activity" has a relationship with the "basic information" such as age, weight, and sex or with the "information regarding physical information" related to body conditions such as vascular function, glucose metabolism, and cognitive function. Further, it is suggested that the "amount of activity" has a relationship with the "information regarding a psychological state" such as stress and mental fatigue.

<Overall Configuration>

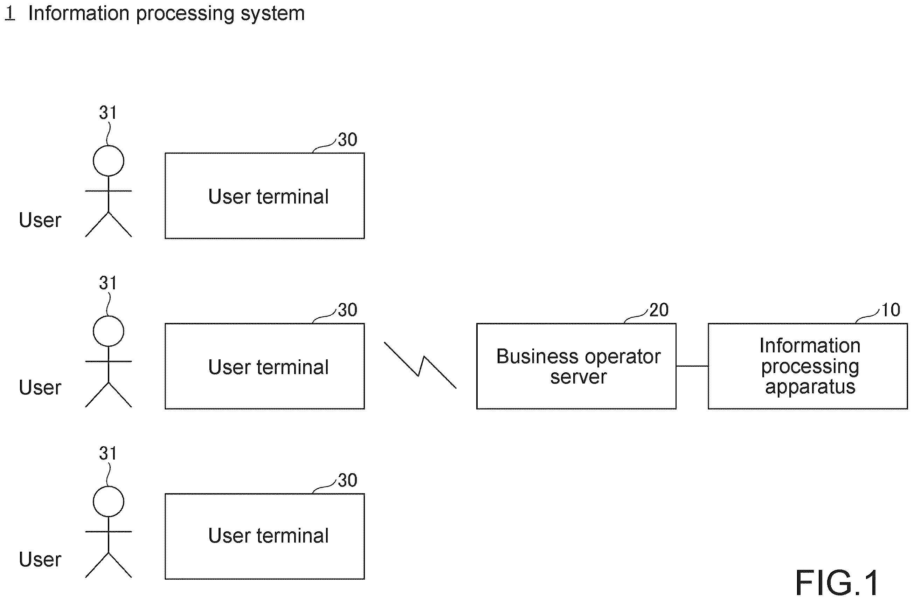

Fig. 1 is an example of the overall configuration according to one embodiment of the present disclosure. An information processing system 1 includes an information processing apparatus 10, a business operator server 20, and a user terminal 30. Note that part or all of the information processing apparatus 10, the business operator server 20, and the user terminal 30 may be implemented in one device. Further, a plurality of information processing apparatuses 10 or a plurality of business operator servers 20 may be provided. This will be described below.

<<Information Processing Apparatus>>

The information processing apparatus 10 is an apparatus for estimating a missing value of body information. The information processing apparatus 10 includes one or more computers. The information processing apparatus 10 can transmit and receive data to and from the business operator server 20 via any network.
Specifically, the information processing apparatus 10 acquires an estimated value of a first attribute by inputting body information in which a value of the first attribute is missing to at least one trained model, and acquires an estimated value of a second attribute by inputting body information in which a value of the second attribute is missing to the at least one trained model, the second attribute being different from the first attribute. Note that the first attribute and the second attribute are preferably body information other than the basic information. Here, the expression of "a value of an attribute is missing" includes at least "a case where a value of an attribute does not exist", "a case where a value of an attribute is missing", "a case where a value of an attribute is not measured", "a case where a value of an attribute is not acquired", "a case where a value of an attribute is not input and there is a missing flag for the value of the attribute", "a case where a value of an attribute is not accessible", or "a case where a value of an attribute is lost".

<<Business Operator Server>>

The business operator server 20 is a server that is managed by a business operator who provides an estimated value of the body information to a user 31. The business operator server 20 includes one or more computers. The business operator server 20 can transmit and receives data to and from the information processing apparatus 10 and the user terminal 30 via any network.
Specifically, the business operator server 20 calls an application programming interface (API) provided by the information processing apparatus 10 in response to a request from the user terminal 30, and receives an estimated value of the body information from the information processing apparatus 10. Note that the business operator server 20 may receive the estimated value of the body information from the information processing apparatus 10 without using the API.
Note that the case where the information processing system 1 includes the business operator server 20 has been described in Fig. 1, but the user terminal 30 may receive the estimated value of the body information from the information processing apparatus 10 without intervention of the business operator server 20. In this case, the information processing system 1 may include the information processing apparatus 10 and the user terminal 30, and the user terminal 30 can directly transmit and receive data to and from the information processing apparatus 10.

<<User Terminal>>

The user terminal 30 is a terminal operated by the user 31. For example, the user terminal 30 is a smartphone, a tablet, a personal computer, or the like. The user terminal 30 can transmit and receive data to and from the business operator server 20 via any network.
Specifically, the user terminal 30 requests the business operator server 20 to estimate a missing value of body information. Further, the user terminal 30 receives an estimated value of the body information from the business operator server 20.
The user terminal 30 transmits the body information in which a value of a first attribute is missing to the information processing apparatus 10 and receives an estimated value of the first attribute from the information processing apparatus 10, and transmits the body information in which a value of a second attribute different from the first attribute is missing to the information processing apparatus 10 and receives an estimated value of the second attribute from the information processing apparatus. Note that the first attribute and the second attribute are preferably body information other than the basic information. Here, the information processing apparatus 10 may acquire the estimated value of the first attribute and the estimated value of the second attribute by using one trained model, or may acquire the estimated value of the first attribute and the estimated value of the second attribute by using a plurality of trained models. In addition, the user terminal 30 receives information of an additional attribute from the information processing apparatus 10, transmits a value of the additional attribute to the information processing apparatus 10, and receives a second estimated value different from the estimated value of the first attribute from the information processing apparatus 10. Note that the additional attribute is an attribute determined by the information processing apparatus 10 on the basis of the body information in which the value of the first attribute is missing. Note that the estimation of the basic information is not excluded.

Note that the number of times that the user terminal 30 requests estimation of a missing value is not limited to a plurality of times as described above, and may be only one time. In this case, the user terminal 30 can transmit the body information in which the value of the first attribute is missing to the information processing apparatus 10 and receive the estimated value of the first attribute from the information processing apparatus 10. The estimated value of the first attribute is calculated using at least one trained model included in the information processing apparatus 10. The at least one trained model is a model that is trained using at least two data sets, and the two data sets include missing attributes different from each other and include at least one common attribute value.

For example, the user terminal 30 can transmit the body information in which the values of some attributes are missing to the information processing apparatus 10 (the business operator server 20 may intervene). Alternatively, body information in which values of some attributes are missing is stored in another apparatus, and the other apparatus can transmit the body information from the other apparatus to the information processing apparatus 10 (the business operator server 20 may intervene) on the basis of an instruction from the user terminal 30. Alternatively, body information in which values of some attributes are missing is stored in the business operator server 20, and the business operator server 20 can transmit the body information from the business operator server 20 to the information processing apparatus 10 on the basis of an instruction from the user terminal 30.

<Functional Blocks>

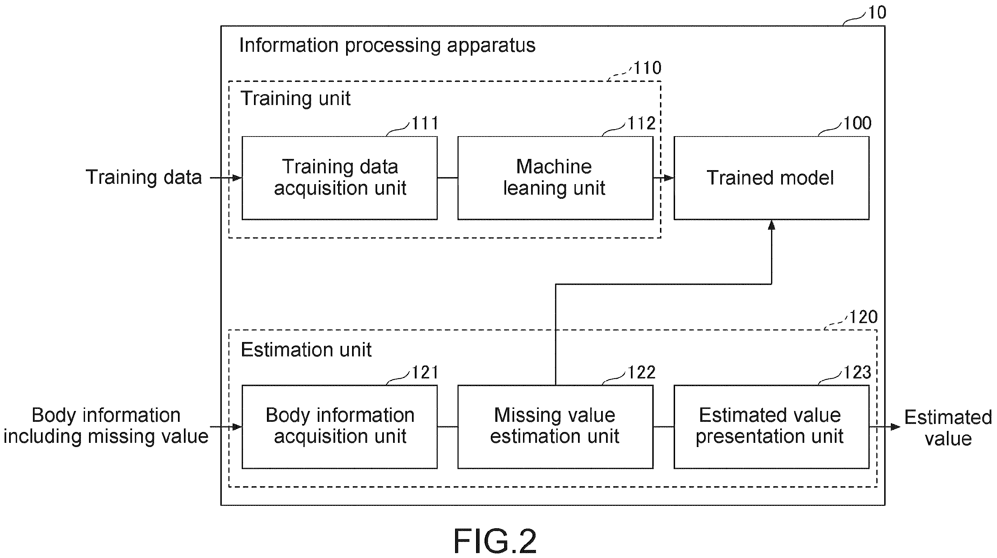

Fig. 2 is a functional block diagram of the information processing apparatus 10 according to one embodiment of the present disclosure. The information processing apparatus 10 includes a training unit 110 and an estimation unit 120.

<<Training Unit>>

The training unit 110 includes a training data acquisition unit 111 and a machine leaning unit 112.

The training data acquisition unit 111 acquires training data of machine learning (specifically, a plurality of pieces of body information).

The machine leaning unit 112 generates a trained model 100 (trains a trained model 100) by machine learning using the training data acquired by the training data acquisition unit 111.

The trained model 100 is a model that outputs estimated values of some attributes by inputting body information in which values of some attributes (note that some attributes may be one attribute or a plurality of attributes) in the attributes of the body information are missing. The trained model 100 is, as an example, a model that can estimate a joint probability distribution (e.g., HI-VAE (https://arxiv.org/pdf/1807.03653.pdf), or TabTransformer (https://arxiv.org/pdf/2012.06678. pdf)). Further, the trained model 100 can employ various methods in outputting an estimated value. Various other machine learning models can also be employed, for example, multiple regression analysis, logistic regression model, a multi-layer perceptron, neural networks such as a convolutional neural network (CNN) and a recurrent neural network (RNN), a support vector machine using any kernel function such as a Gaussian kernel, random forests modeled as regression trees, models utilizing hidden Markov models, statistical models, and stochastic models. Further, a model that performs comprehensive determination by combining various models can also be employed. Of those, a model that can estimate a joint probability distribution (e.g., HI-VAE, TabTransformer) is particularly preferable.

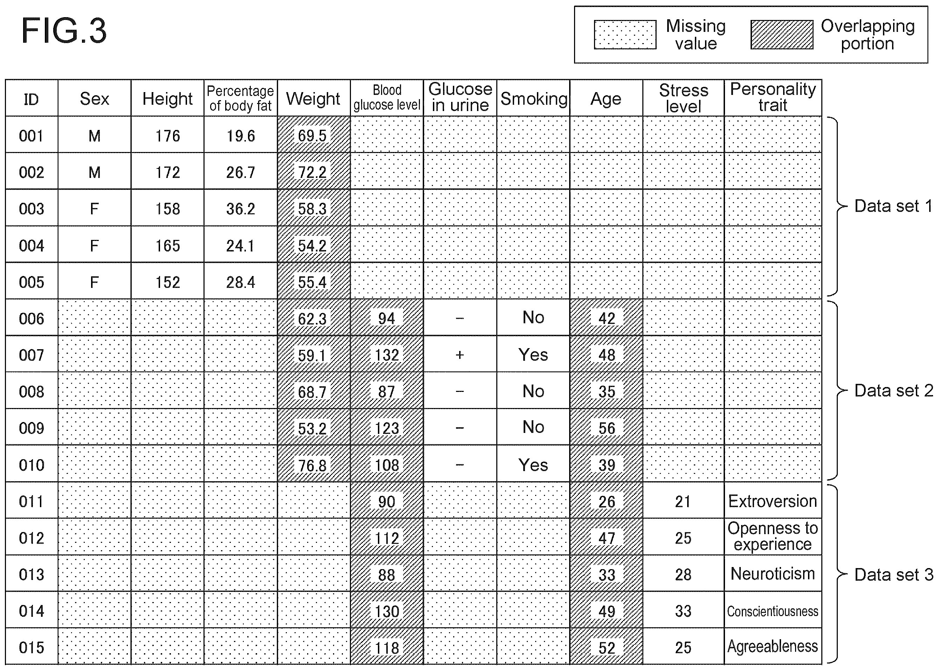

Here, the training data will be described. The training data is preferably a plurality of pieces of body information. At least one trained model 100 is a model that is trained using at least two data sets, and the two data sets include missing attributes different from each other and include at least one common attribute value. Hereinafter, the training data will be described in detail with reference to Fig. 3. Note that the expression of "including missing attributes different from each other" typically means that two or two or more data sets include differences in a list of attribute items of a plurality of pieces of attribute data included in each data set. Specifically, this means that at least some attribute items do not overlap between the list of attribute items held by one data set and the list of attribute items held by another data set.

Fig. 3 is a diagram for describing the training data according to one embodiment of the present disclosure. In one embodiment of the present disclosure, the training data includes a plurality of data sets (note that each data set includes one or more pieces of body information; in the example of Fig. 3, a data set 1 including ID: 001 to ID: 005 (relationship data between weight and percentage of body fat, sex, and height), a data set 2 including ID: 006 to ID: 010 (relationship data between weight and blood glucose level, glucose in urine, smoking, and age), and a data set 3 including ID: 011 to ID: 015 (relationship data between age and stress level, stress level and personality traits, and blood glucose level)). Note that the body information indicated by the respective IDs may be body information of a plurality of different persons, or may be body information of the same person acquired at different periods or different locations (different facilities or the like).

A common attribute of the data set 1 (ID: 001 to 005) and the data set 2 (ID: 006 to 010) is weight (basic information). In the data set 1, the information of blood glucose level, glucose in urine, smoking, age, stress level, and personality traits are missing, and in the data set 2, the information of sex, height, percentage of body fat, stress level, and personality traits are missing. Further, common attributes of the data set 2 and the data set 3 (ID: 011 to 015) are age (basic information) and blood glucose level (information regarding body). In the data set 3, the information of sex, height, percentage of body fat, weight, glucose in urine, and smoking are missing. For example, a training method using HI-VAE to be described below in <training method when using HI-VAE> is executed on the basis of those data sets 1 to 3, so that a trained model based on ID: 001 to 015 can be generated. Note that one training model is created by combining all of the data sets 1 to 3 in the above example, but the present technology is not limited to this example, and a desired training model may be obtained by combining the data sets 1 to 3 in stages.

The plurality of pieces of body information that are training data include body information in which values of different attributes are missing. Referring to Fig. 3, the data set 1 includes missing values of blood glucose level, glucose in urine, smoking, age, stress level, and personality traits, the data set 2 includes missing values of sex, height, percentage of body fat, stress level, and personality traits, and the data set 3 includes missing values of sex, height, percentage of body fat, weight, glucose in urine, and smoking.

The plurality of pieces of body information that are training data include body information including at least one same attribute value. Referring to Fig. 3, the data set 1 and the data set 2 include the values of the same attribute (in the example of Fig. 3, weight as overlapping portion), and the data set 2 and the data set 3 include the values of the same attributes (in the example of Fig. 3, blood glucose level and age as overlapping portions). An attribute (i.e., weight of Fig. 3) including values in both a certain data set (e.g., data set 2 of Fig. 3) and another data set (e.g., data set 1 of Fig. 3), and attributes (i.e., blood glucose level and age of Fig. 3) including values in both the certain data set (e.g., data set 2 of Fig. 3) and still another data set (e.g., data set 3 of Fig. 3) may be different attributes.

For example, the data sets are data sets acquired in different situations (e.g., a medical checkup and a questionnaire). Further, for example, the data sets are data sets in which values of different attributes are acquired in the same situation (e.g., a basic test item and a specific test item of a medical checkup). Further, the data sets may be body information acquired at different locations or may be acquired at different periods. Further, body information of the same person may be included between the data sets.

As described above, in this embodiment, the trained model 100 is generated using the training data obtained by integrating a plurality of data sets in which values of different attributes are missing, which makes it possible to increase the body information that can be estimated by the trained model 100. Further, in this embodiment, since data sets in which values of different attributes are missing can be integrated, it is possible to effectively use, as one training data, the results of a plurality of medical checkups including different examination items held by respective facilities.

Here, when a model is trained by combining different data sets, at least one attribute (hereinafter, referred to as a common attribute) that is commonly included in a plurality of data sets needs to exist. For example, weight, blood glucose level, and age in Fig. 3 correspond to the common attributes. The common attributes exist, which makes it possible to improve a prediction accuracy also in the case where an attribute specific to a certain data set is predicted from an attribute specific to a different data set.

Here, the attribute including values in common among the data sets, i.e., "common attribute", preferably includes one or two or more attributes selected from the "basic information". Further, it is preferable that the "common attribute" further includes one or two or more attributes selected from the "information regarding a physical condition".

Note that, after the training data is randomly lost, an error function for restoring data may be minimized. If the data is artificially lost, more robust estimation is made possible.

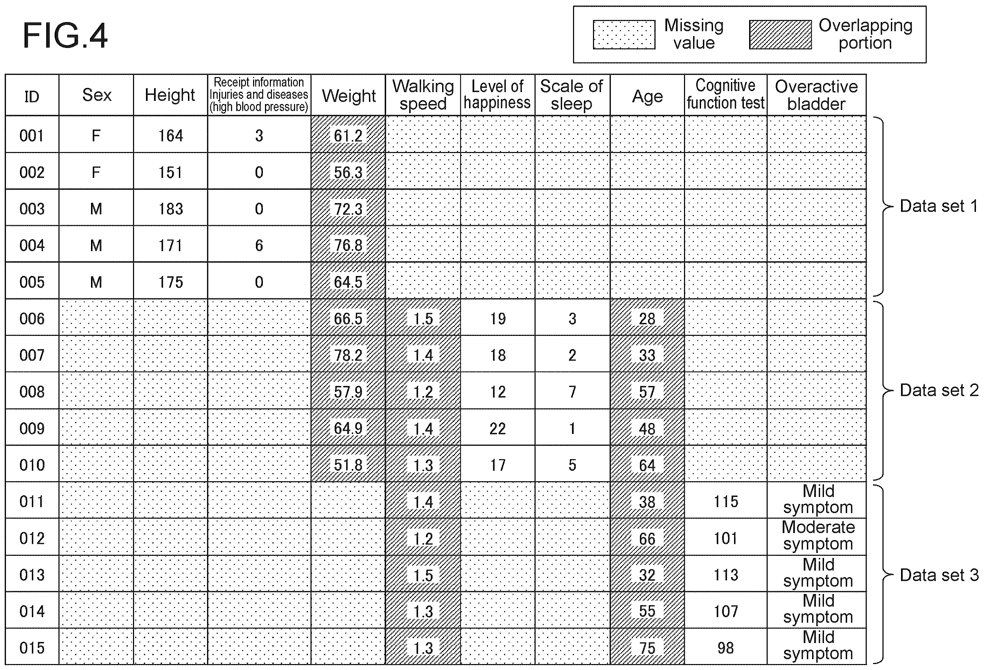

Note that the training data of Fig. 3 is merely an example, and it is possible to use training data of discretionary body information such as the training data described in Fig. 4.

If a neural network is used as a model, a technique commonly used for a neural network (e.g., Adam, Momentum SGD) can be used for optimization. For example, if HI-VAE (https://arxiv.org/pdf/1807.03653.pdf) is used as a model, there are hyperparameters such as the dimensions of hidden variables, and they can be combined with hyperparameter searching to determine what is suitable for prediction.

<Training Method When Using HI-VAE>

Hereinafter, an example of a training method when HI-VAE is used as a model will be described.

    1. Training data including a plurality of data sets is prepared.

        (a) As an example, data of IDs included in training data are four-dimensional vectors (attributes A to D).

(1) Example) attribute A: 10, attribute B: 8, attribute C: missing, attribute D: 5

(b) Any of the following values is put in a part in which a value is missing (in the above example, attribute C) in the four-dimensional vectors. Note that the missing part may differ between the data of the respective IDs.

(1) NULL (that may be a predetermined symbol)
(2) 0
(3) Numerical value determined on the basis of the value of the attribute included in the training data (as an example, an average value etc.)

(c) If 0 of (2) is used, examples of the vectors are as follows.

(1) Example) attribute A: 10, attribute B: 8, attribute C: 0, attribute D: 5

2. Some attributes of the training data are erased.

(a) As an example, the value of the attribute D is erased (the value is replaced with 0).

(1) Example) attribute A: 10, attribute B: 8, attribute C: 0, attribute D: 0

(b) The attribute to be erased may be randomly set for the vectors of the respective IDs.

3. A plurality of vectors corresponding to the respective IDs generated in the above "2" are input to HI-VAE, and a plurality of output vectors are obtained.

(a) In the output vectors corresponding to the example described above, not only an estimated value of the attribute D but also estimated values of the other attributes A, B, and C are output. In other words, in the output vectors, the values the attribute A, the attribute B, and the attribute C are also replaced with the estimated values. An example of the output vectors is as follows.

(1) Example) attribute A: 11, attribute B: 7, attribute C: 3, attribute D: 4

4. The parameters of the model are updated such that the difference (error) between the vectors generated in the above "2" and the vectors output by the model in the above "3" corresponding thereto becomes small.

(a) Here, in the output vectors of the example described above, the attribute C is a missing part that does not include an original value (a part having no correct answer), and thus is not considered in calculation of an error.

<Estimation Method When Using HI-VAE>

Hereinafter, an example of an estimation method when HI-VAE is used as a model will be described.

1. An input vector is generated using body information (with some attributes missing) received from the user terminal 30.

(a) Any of the following values is put in a missing attribute value.

(1) NULL (that may be a predetermined symbol)
(2) 0
(3) Numerical value determined on the basis of the value of the attribute included in training data (as an example, an average value etc.)

(b) There may be a plurality of attributes including missing values.

2. The vector in "1" is input to a trained model (HI-VAE), and an output vector is obtained.

(a) In the output vector, an estimated value is put in the missing part of the attribute, and also estimated values are put in other non-missing attributes.

3. The estimated value is presented to the user terminal 30.

(a) Here, the information processing apparatus 10 may present only the estimated value of the missing attribute in "1" to the user terminal 30.
(b) Further, the information processing apparatus 10 may combine the estimated value of the missing attribute in "1" and the body information received from the user terminal 30 in "1" to present it to the user terminal 30.

Now Fig. 2 will be described again.

<<Estimation Unit>>

The estimation unit 120 includes a body information acquisition unit 121, a missing value estimation unit 122, and an estimated value presentation unit 123.

The body information acquisition unit 121 may acquire body information in which values of some attributes (note that some attributes may be one attribute or a plurality of attributes) in the attributes of the body information are missing. Specifically, the body information acquisition unit 121 acquires each attribute name and a value of each attribute (note that it is Null or the like if the value is missing).

The missing value estimation unit 122 acquires estimated values (e.g., expected values or variances) of some attributes by inputting the body information acquired by the body information acquisition unit 121 (i.e., body information in which values of some attributes in the attributes of the body information are missing) to the trained model 100. The missing value estimation unit 122 may acquire at least one estimated value with respect to a missing attribute, or may acquire a value having a predetermined range as an estimated value. Further, the missing value estimation unit 122 can acquire the confidence coefficient of the estimated value. Note that the missing value estimation unit 122 may acquire an estimated value using a trained model selected from a plurality of trained models. Further, the missing value estimation unit 122 may perform estimation processing using a plurality of trained models, and use the estimated value having the highest confidence coefficient as an estimated value to be presented to the user. In addition, the missing value estimation unit 122 may select a trained model to be used for estimation from among a plurality of trained models on the basis of an instruction from the user terminal 30.

Note that the missing value estimation unit 122 may estimate other information (e.g., information on whether or not an active ingredient Q is effective) by inputting the estimated value to a predetermined calculation formula determined in advance or to a machine learning model that is separately trained.

For example, in HI-VAE, not only the estimation as a point but also the estimation as a distribution can be performed in the estimation. When HI-VAE is trained, a distribution is determined in advance for each attribute. For example, the distribution may be a Gaussian distribution (e.g., taking a continuous value, such as weight), a categorical distribution (e.g., taking a discrete value, such as the presence or absence of a disease of a person), a Poisson distribution (e.g., taking a natural number, such as the number of times of suffering from a disease), a lognormal distribution (e.g., taking a positive real number, such as BMI, and also having an asymmetric distribution), and the like. On the basis of the distribution thus determined in advance, the parameter of the distribution is returned in the estimation. For example, in the case of the Gaussian distribution, mean and variance parameters are returned. At that time, it is possible to estimate how much the value estimated using the variance parameter can be shifted. For example, if the mean of the estimated values regarding the weight is 60 and the variance is 1, this estimation indicates that "the probability (confidence coefficient) in which the actual weight falls within 60 kg $\pm$ 2 kg is 95% or more." Similar calculation in confidence intervals can be performed for other distributions (i.e., the estimated value is a value having a predetermined range).

For example, it is possible to provide a notification such as "the attribute A can be estimated with higher accuracy by additionally providing the data of an attribute X in addition to the measured data of an attribute." A method of using several indices is conceivable for recommendation of the attribute X. Here, a method of using an information gain of HI-VAE will be described as an example. The information gain is defined by the following expression.

$$\alpha_{ES}(x; D_n) := H(x^* | D_n) - E_{y | D_n, x} H(x^* | D_n \cup \{(x, y)\})$$

... Mathematical expression (1)

Here, $x^*$ corresponds to the attribute A, x corresponds to a candidate attribute, y corresponds to an estimated value of the candidate attribute, $D_n$ corresponds to training data, $H(x | D_n) = -\int p(x | D_n) \log p(x | D_n) dx$ corresponds to an entropy of a posterior distribution $p(x | D_n)$. Further, E represents an expected value, and $\alpha ES$ represents an expected value of the information gain.

The first term in this expression represents the uncertainty of $x^*$ under the observation of $D_n$, and represents that estimation is more difficult as the value becomes higher. The second term in this expression represents the uncertainty of x* when "a value of y is temporarily observed for an unobserved attribute x" in addition to D_n. In other words, it can be interpreted that subtracting the second term from the first term evaluates how much the uncertainty of x* is reduced when x is added. Although the entropy and the expected value appearing in the Mathematical expression (1) generally have no analytical solutions, an approximate solution can be obtained by using a Monte Carlo method or the like. Calculating this information gain for the candidate attribute and recommending one including the highest value make it possible to recommend an attribute that reduces the uncertainty to a largest extent when x* is estimated. The estimated value presentation unit 123 can prompt the user to provide the value of the attribute (additional attribute) by presenting the information of the attribute that reduces the uncertainty to the largest extent to the user. The missing value estimation unit 122 can further enhance the estimation accuracy of the attribute A by executing the estimation processing again using the value of the additional attribute received from the user. Note that the attribute A may be determined on the basis of an instruction from the user, or a predetermined item may be set as the attribute A.

The estimated value presentation unit 123 presents the estimated values of some attributes estimated by the missing value estimation unit 122. The estimated value presentation unit 123 may present not only the estimated values of some attributes but also the whole of the body information including the estimated values. Note that the estimated value presentation unit 123 can also provide information related to the body information (e.g., a service, a commodity, or the like) on the basis of the estimated values of the attributes. Here, the estimated value presentation unit 123 may acquire the information regarding the body information by inputting the estimated value to a predetermined calculation formula determined in advance or to a machine learning model that is separately trained. Further, the estimated value presentation unit 123 may acquire the information regarding the body information by using body information other than the estimated value.

Note that after acquiring the estimated value, the information processing apparatus 10 can delete the body information in which the value of the attribute is missing (i.e., the body information acquired from the user) and the estimated body information from a storage device (memory) of the information processing apparatus 10. This makes it possible to prevent the user's body information from leaking, and possible for the user to use the information processing apparatus 10 with a sense of security. Further, the information processing system 1 may delete the body information in which the value of the attribute is missing and the estimated body information not only from the information processing apparatus 10 but also from other apparatuses (such as the business operator server 20). Further, the information processing system 1 may delete the body information in which the value of the attribute is missing and the estimated body information from all the apparatuses other than the user terminal 30.

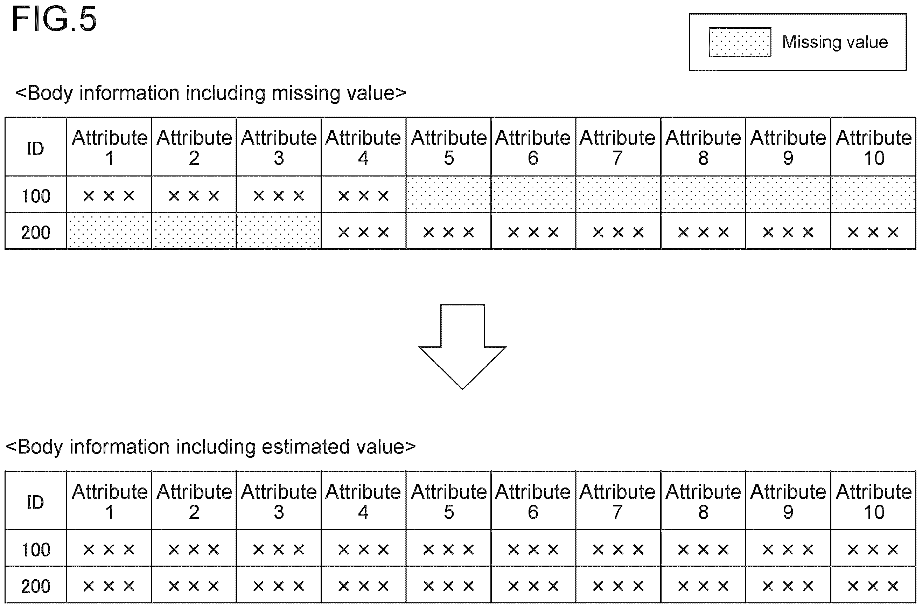

Fig. 5 is a diagram for describing body information including a missing value and body information including an estimated value according to one embodiment of the present disclosure (note that xxx in the drawing indicates determination, score, numerical value, and classification information).

The <body information including a missing value> in the upper part of Fig. 5 is body information in which values of some attributes (note that some attributes may be one attribute or a plurality of attributes) in the attributes of the body information are missing. Referring to Fig. 5, in body information of ID: 100, values of the attributes 5 to 10 are missing. Further, in body information of ID: 200, values of the attributes 1 to 3 are missing. When the <body information including a missing value> is input to the trained model 100, the missing values of the attributes are estimated.

The <body information including an estimated value> in the lower part of Fig. 5 is body information including a value estimated in the trained model 100. Referring to Fig. 5, in the body information of ID: 100, the missing values of the attributes (in the example of Fig. 5, attribute 5 to attribute 10) are estimated. Further, in the body information of ID: 200, the missing values of the attributes (in the example of Fig. 5, attribute 1 to attribute 3) are estimated.

Thus, in one embodiment of the present disclosure, body information in which some attributes include missing values can be received, and body information in which the missing values are replaced with estimated values can be returned.

Note that the estimated value presentation unit 123 can present the estimated body information in the form of table data (e.g., a table structure as shown in Fig. 5). For example, the estimated value presentation unit 123 can present an estimated attribute value in the body information in a different display form (e.g., change the display form on the screen, such as changing the color of only the estimated value, changing the font, emphasizing, and changing the size). For example, the estimated value presentation unit 123 can present the estimated attribute value in a display form according to the confidence coefficient thereof (e.g., the display form can be made different between a high confidence coefficient and a low confidence coefficient). Further, the estimated value presentation unit 123 may present an index representing the confidence coefficient in addition to the body information.

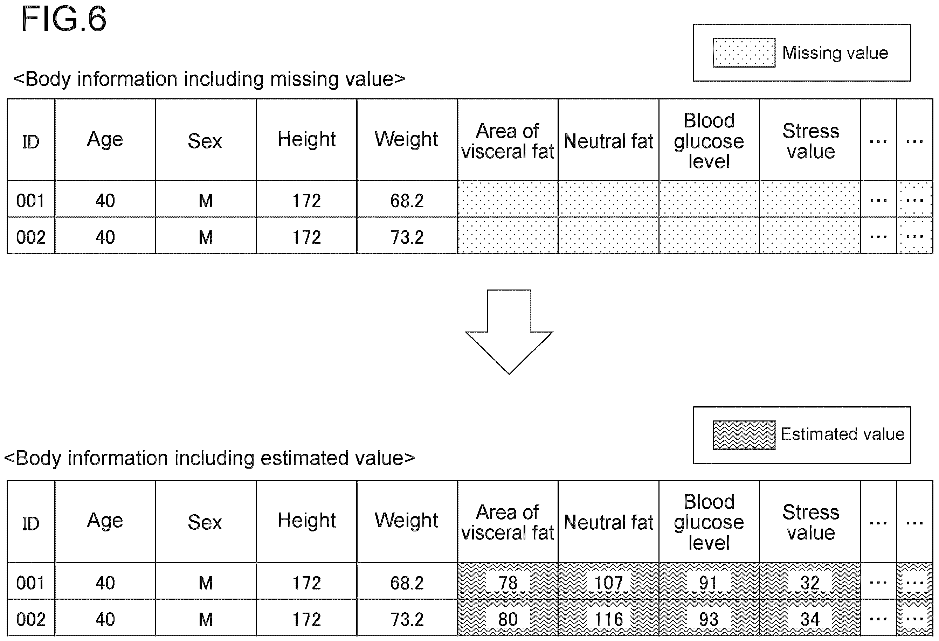

Fig. 6 is a diagram showing an estimation example according to one embodiment of the present disclosure. As shown in <body information including a missing value> in Fig. 6, when body information in which the values of some attributes (in the example of Fig. 6, "area of visceral fat", "neutral fat", "blood glucose level", and "stress level") in the attributes of the body information are missing is input to the trained model 100, the missing values of the attributes are estimated as shown in <body information including an estimated value> in Fig. 6.

Hereinafter, an estimation example of the body information will be described. Note that a plurality of estimation examples in the following estimation examples may be combined and implemented.

<<Estimation Example 1>>

The missing value estimation unit 122 can estimate a missing attribute value (e.g., estimate a current value). For example, the missing value estimation unit 122 can estimate a probability of a certain disease or estimate a value of a blood test without blood sampling.

<<Estimation Example 2>>

The missing value estimation unit 122 can convert the value of the attribute (note that the value may include not only an estimated value but also a measured value) into an index that is easily understood by the user, and can show it. For example, the missing value estimation unit 122 can calculate a health age, a vascular age, a physical strength age, and the like on the basis of the value of the attribute. Note that the missing value estimation unit 122 may calculate the health age, the vascular age, the physical strength age, and the like by inputting an estimated value to a predetermined calculation formula determined in advance or inputting the estimated value to a machine learning model that is separately trained.

<<Estimation Example 3>>

The missing value estimation unit 122 can show, when a user selects an attribute to be estimated, an attribute to be measured in order to increase the confidence coefficient in estimating the value of the attribute. For example, the missing value estimation unit 122 can show an attribute to be measured in order to narrow a confidence interval in estimating a value of a certain attribute (e.g., what is to be measured in order to narrow a confidence interval when an estimated probability of diabetes is 40 to 800). For example, it can be implemented by the method using the information gain of HI-VAE described above.

<<Estimation Example 4>>

The missing value estimation unit 122 can extract an attribute that changes in conjunction with the change of a value of a certain attribute. For example, if an attribute that changes in conjunction with lowering of a value of blood glucose level is extracted, an attribute that is effective for lowering the blood glucose level can be presented to the user. Here, a target attribute such as the blood glucose level may be determined on the basis of an instruction from the user terminal 30.

<<Estimation Example 5>>

The missing value estimation unit 122 can input an assumed value to a certain attribute to estimate a value of another attribute. In other words, when an assumed value is input to a certain attribute, the missing value estimation unit 122 can show what changes are present in other attributes (e.g., if the weight is 5 kg lighter, the probability of diabetes is slightly lower). In this way, establishing a causal hypothesis with an attribute that changes with a change of a certain attribute and verifying it in a clinical trial or the like makes it possible to lead to the discovery of a new relationship between attributes that appear irrelevant to each other at first glance. The missing value estimation unit 122 may input an assumed value to a certain attribute on the basis of an instruction from the user terminal 30, and transmit the result to the user terminal 30.

<<Estimation Example 6>>

The missing value estimation unit 122 can perform estimation using a trained model (e.g., a model of a smoker and a model of a nonsmoker) generated on the basis of a specific attribute. The missing value estimation unit 122 may store a plurality of trained models trained using training data based on attributes in the storage device, and switch between the trained models to be used for the estimation on the basis of the attribute of body information to be input. Note that the attribute may be based on basic information. The missing value estimation unit 122 may determine an attribute to be used for switching the trained model on the basis of an instruction from the user terminal 30.

<<Estimation Example 7>>

The missing value estimation unit 122 can acquire a representative value in a certain attribute as a value of a probability distribution, and can indicate a position on a probability distribution of user's values. For example, the missing value estimation unit 122 can acquire a standard value in a certain attribute and indicate a relative index (e.g., a value of the same sex and age as the user him/herself, and a position on a probability distribution of the user's results) .

<Processing Method>

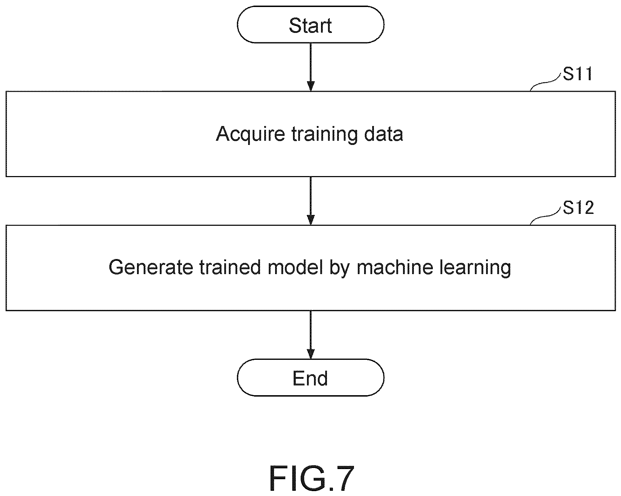

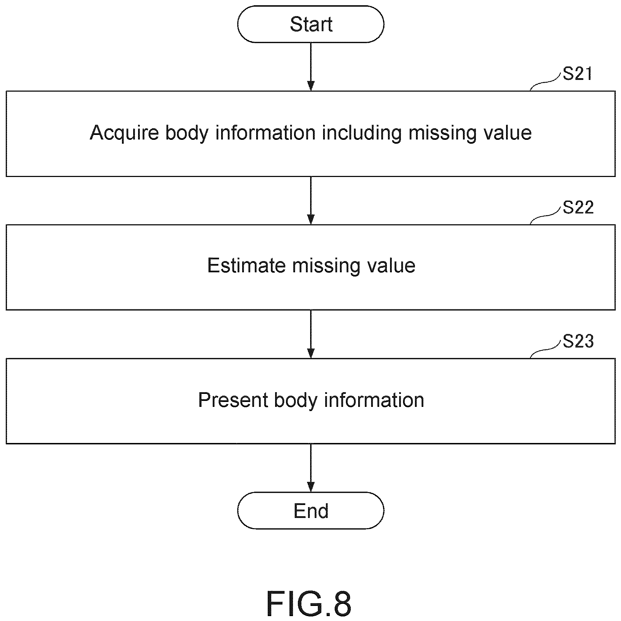

Hereinafter, training processing will be described with reference to Fig. 7, and inference processing will be described with reference to Fig. 8.

<<Training Processing>>

Fig. 7 is a flowchart of training processing according to one embodiment of the present disclosure.
In Step 11 (S11), the training data acquisition unit 111 acquires training data of machine learning (specifically, a plurality of pieces of body information).
In Step 12 (S12), the machine leaning unit 112 generates a trained model 100 by the machine learning using the training data acquired in S11.

<<Inference Processing>>

Fig. 8 is a flowchart of estimation processing according to one embodiment of the present disclosure.
In Step 21 (S21), the body information acquisition unit 121 acquires body information in which values of some attributes (note that some attributes may be one attribute or a plurality of attributes) in the attributes of the body information are missing.
In Step 22 (S22), the missing value estimation unit 122 acquires estimated values of some attributes by inputting the body information acquired in S21 (i.e., body information in which values of some attributes in the attributes of the body information are missing) to the trained model 100.
In Step 23 (S23), the estimated value presentation unit 123 presents the estimated values of some attributes that has been estimated in S22. Here, the estimated value presentation unit 123 may present the body information acquired in S21 and the estimated values acquired in S22 in a combined manner, or may present only the estimated values acquired in S22.

<Effects>

As described above, in the embodiment of the present disclosure, if the body information in which values of some attributes are missing is input to the trained model, the values of some of the attributes can be estimated, so that even if the values of all of the attributes are not measured, the values of all of the attributes can be obtained.
Part or all of each apparatus (information processing apparatus 10 or user terminal 30) in the embodiment described above may be configured by hardware, or may be configured by information processing of software (programs) executed by a central processing unit (CPU), a graphics processing unit (GPU), or the like. When part or all of each apparatus is configured by information processing of software, software for implementing at least part of the functions of each apparatus in the embodiment described above may be stored in a non-transitory storage medium (non-transitory computer-readable medium) such as a compact disc-read only memory (CD-ROM) or a universal serial bus (USB) memory, and may be read by the computer, so that the information processing of the software may be executed. Further, the software may be downloaded via a communication network. In addition, all or part of the processes of the software may be implemented in a circuit such as an application specific integrated circuit (ASIC) or a field programmable gate array (FPGA), so that the information processing by the software may be executed by hardware.
The storage medium for storing the software may be a removable storage medium such as an optical disc, or may be a fixed storage medium such as a hard disk or a memory. Further, the storage medium may be provided inside the computer (main storage device, auxiliary storage device, or the like), or may be provided outside the computer.

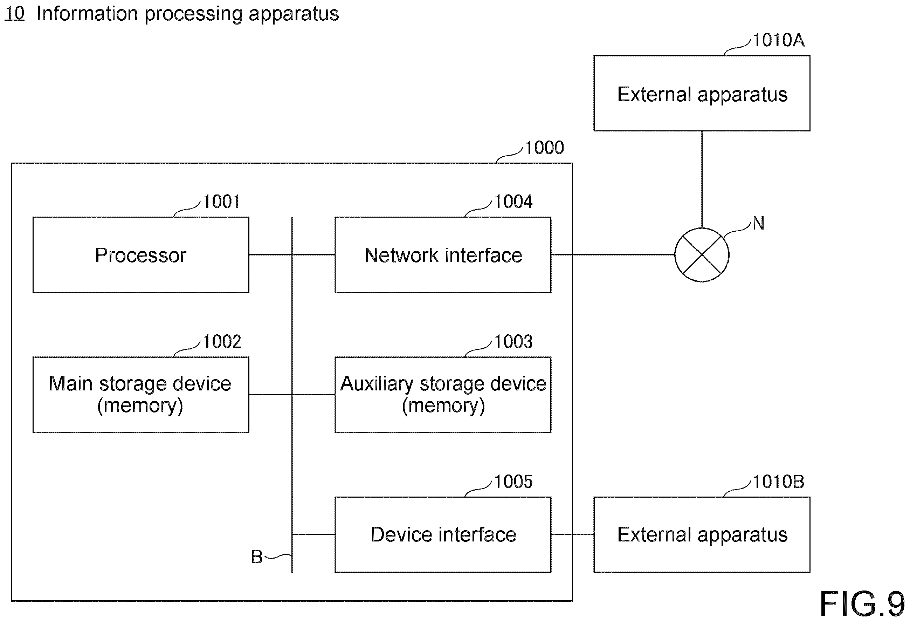

Fig. 9 is a block diagram showing an example of a hardware configuration of each apparatus (information processing apparatus 10 or user terminal 30) in the embodiment described above. Each apparatus includes, for example, a processor 1001, a main storage device 1002 (memory), an auxiliary storage device 1003 (memory), a network interface 1004, and a device interface 1005, which may be implemented as a computer 1000 connected via a bus B.
The computer 1000 of Fig. 9 includes each constituent element one by one, but may include a plurality of the same constituent elements. Further, although Fig. 9 shows one computer 1000, the software may be installed in a plurality of computers, and each of the plurality of computers may execute the same or different part of the processing of the software. In this case, the form of distributed computing may be provided, in which each computer performs communication and processing via the network interface 1004 or the like. In other words, each apparatus (information processing apparatus 10 or user terminal 30) in the embodiment described above may be configured as a system that implements functions by one or more computers executing instructions stored in one or more storage devices. Further, the following configuration may also be provided: the information transmitted from a terminal may be processed by one or more computers provided on the cloud, and a processing result thereof may be transmitted to the terminal.

Various operations of each apparatus (information processing apparatus 10 or user terminal 30) in the embodiment described above may be executed in parallel processing using one or more processors or using a plurality of computers via a network. Further, various operations may be distributed to a plurality of operation cores in the processor and executed in parallel processing. Further, part or all of the processes, means, and the like of the present disclosure may be implemented by at least one of a processor or a storage device provided on a cloud capable of communicating with the computer 1000 via a network. As described above, each apparatus in the embodiment described above may be in the form of parallel computing by one or more computers.

The processor 1001 may be an electronic circuit (processor circuit, processing circuit, processing circuitry, CPU, GPU, FPGA, ASIC, or the like) that performs at least one of computer control or operation. Further, the processor 1001 may be any of a general-purpose processor, a special-purpose processing circuit designed to execute a specific operation, or a semiconductor device including both a general-purpose processor and a special-purpose processing circuit. Further, the processor 1001 may include an optical circuit, or may include an arithmetic function based on quantum computing.

The processor 1001 may perform arithmetic processing on the basis of software or data input from each device or the like in the internal configuration of the computer 1000 and may output an arithmetic result or a control signal to each device or the like. The processor 1001 may control the constituent elements constituting the computer 1000 by executing an operating system (OS) of the computer 1000, an application, or the like.

Each apparatus (information processing apparatus 10 or user terminal 30) in the embodiment described above may be implemented by one or more processors 1001. Here, the processor 1001 may refer to one or more electronic circuits disposed on one chip, or may refer to one or more electronic circuits disposed on two or more chips or two or more devices. When a plurality of electronic circuits are used, each electronic circuit may communicate with each other by wire or wirelessly.

The main storage device 1002 may store instructions to be executed by the processor 1001, various types of data, and the like, and information stored in the main storage device 1002 may be read by the processor 1001. The auxiliary storage device 1003 is a storage device other than the main storage device 1002. Note that those storage devices are assumed to mean any electronic component capable of storing electronic information, and may be semiconductor memories. The semiconductor memory may be either a volatile memory or a non-volatile memory. The storage device for storing various types of data or the like in each apparatus (information processing apparatus 10 or user terminal 30) in the embodiment described above may be implemented by the main storage device 1002 or the auxiliary storage device 1003, or may be implemented by a built-in memory built in the processor 1001. For example, the storage unit in the embodiment described above may be implemented by the main storage device 1002 or the auxiliary storage device 1003.

If each apparatus (information processing apparatus 10 or user terminal 30) in the embodiment described above includes at least one storage device (memory) and at least one processor connected (coupled) to the at least one storage device, at least one processor may be connected to one storage device. Further, at least one storage device may be connected to one processor. Further, the following configuration may be included: at least one of a plurality of processors is connected to at least one storage device of a plurality of storage devices. Further, this configuration may be achieved by storage devices and processors included in a plurality of computers. In addition, a configuration in which a storage device is integrated with a processor (e.g., a cache memory including an L1 cache and an L2 cache) may be included.

The network interface 1004 is an interface for connecting to a communication network N wirelessly or by wires. The network interface 1004 only needs to use a suitable interface such as one conforming to an existing communication standard. The network interface 1004 may exchange information with an external apparatus 1010A connected via the communication network N. Note that the communication network N may be any one of a wide area network (WAN), a local area network (LAN), a personal area network (PAN), and the like, or a combination thereof, and only needs to be one that exchanges information between the computer 1000 and the external apparatus 1010A. Examples of the WAN include the Internet. Examples of the LAN include IEEE 802.11 and Ethernet (registered trademark), and examples of the PAN include Bluetooth (registered trademark) and near field communication (NFC).

The device interface 1005 is an interface such as a USB that is directly connected to an external apparatus 1010B.

The external apparatus 1010A is an apparatus connected to the computer 1000 via a network. The external apparatus 1010B is an apparatus directly connected to the computer 1000.

The external apparatus 1010A or the external apparatus 1010B may be an input apparatus as an example. The input apparatus is, for example, a device such as a camera, a microphone, a motion capture, various sensors, a keyboard, a mouse, or a touch panel, and provides acquired information to the computer 1000. Further, the input apparatus may be a device including an input unit, a memory, and a processor, such as a personal computer, a tablet terminal, or a smartphone.

Further, the external apparatus 1010A or the external apparatus 1010B may be an output apparatus as an example. The output apparatus may be, for example, a display apparatus such as a liquid crystal display (LCD) or an organic electro luminescence (EL) panel, or may be a speaker or the like that outputs sound or the like. Further, the output apparatus may be a device including an output unit, a memory, and a processor, such as a personal computer, a tablet terminal, or a smartphone.

Further, the external apparatus 1010A or the external apparatus 1010B may be a storage device (memory). For example, the external apparatus 1010A may be a network storage or the like, and the external apparatus 1010B may be storage such as an HDD.

Further, the external apparatus 1010A or the external apparatus 1010B may be an apparatus having a function of part of the constituent elements of each apparatus (information processing apparatus 10 or user terminal 30) in the embodiment described above. In other words, the computer 1000 may transmit part or all of the processing results to the external apparatus 1010A or the external apparatus 1010B, or may receive part or all of the processing results from the external apparatus 1010A or the external apparatus 1010B.

In this specification (including claims), if the expression "at least one of (one of) a, b, and c" or "at least one of (one of) a, b, or c" (including similar expressions) is used, it includes either a, b, c, a-b, a-c, b-c, or a-b-c. Further, a plurality of instances for any element, such as a-a, a-b-b, and a-a-b-b-c-c, may be included. In addition, it also includes adding other elements other than the listed elements (a, b, and c), such as including d as in a-b-c-d.

In this specification (including claims), if the expression such as "with data used as input/using data/on the basis of data/according to data/in accordance with data" (including similar expressions) is used, unless otherwise specified, a case where the data itself is used, and a case where one obtained by performing some processing on data (e.g., one with noise being added, one normalized, a feature amount extracted from the data, or an intermediate expression of the data) is used are included. Further, if it is described that a certain result is obtained "with data used as input/using data/on the basis of data/according to data/in accordance with data" (including similar expressions), unless otherwise specified, a case where a result is obtained only on the basis of the data, and a case where a result is obtained by being affected by other data other than the data, factors, conditions, and/or states are included. Further, if it is described that "data is output" (including similar expressions), unless otherwise specified, a case where the data itself is used as an output, and a case where one obtained by performing some processing on the data (e.g., one with noise being added, one normalized, a feature amount extracted from the data, or an intermediate expression of the data) is used as an output are included.

In this specification (including claims), if the terms "connected" and "coupled" are used, they are intended as non-limiting terms, including any of direct connection/coupling, indirect connection/coupling, electrical connection/coupling, communicative connection/coupling, operative connection/coupling, physical connection/coupling, and the like. Those terms should be interpreted as appropriate depending on the context in which the terms are used, but a connection/coupling form that is not intentionally or naturally excluded should be interpreted as being included in the terms and as not being limited.

In this specification (including claims), if the expression "A configured to B" is used, it may include that the physical structure of the element A has a configuration capable of performing the operation B and that a permanent or temporary setup (setting/configuration) of the element A is configured/set to actually perform the operation B. For example, if the element A is a general-purpose processor, the processor only needs to have a hardware configuration capable of executing the operation B and to be set (configured) to actually execute the operation B by setting a permanent or temporary program (instruction). Further, if the element A is a dedicated processor, a dedicated arithmetic circuit, or the like, a circuit structure or the like of the processor only needs to be constructed (implemented) so as to actually execute the operation B regardless of whether or not a control instruction and data are actually attached thereto.

In this specification (including claims), the terms that mean inclusion or possession (e.g., "comprising/including", "having", etc.) are used, they are intended as open-ended terms, including the inclusion or possession of an object other than a target indicated by the objective of the terms. If an objective of a term that means inclusion or possession is an expression that does not specify a quantity or suggests a singular number (expressing with "a" or "an" as an article), the expression should be interpreted as not being limited to a specific number.

In this specification (including claims), if an expression such as "one or more", "at least one," or the like is used in a certain location, and an expression that does not specify a quantity or suggests a singular number (expressing with "a" or "an" as an article) is used in another location, the latter expression is not intended to mean "one". In general, the expression that does not specify a quantity or suggests a singular number (expressing with "a" or "an" as an article) should be interpreted as not necessarily being limited to a specific number.

In this specification, if it is described that a specific effect (advantage/result) is obtained with respect to a specific configuration of an embodiment, it should be understood that the effect can also be obtained with respect to another embodiment or a plurality of embodiments having the configuration, unless otherwise specified. However, it should be understood that the presence or absence of the effect generally depends on various factors, conditions, and/or states, and the effect is not necessarily obtained by the configuration. The effect is merely obtained by the configuration described in the embodiment when various factors, conditions, and/or states are satisfied, and the effect is not necessarily obtained in the invention according to the claims that define the configuration or similar configuration.

In this specification (including claims), if the terms such as "maximize"/"maximization" are used, they include determining a global maximum value, determining an approximation of a global maximum value, determining a local maximum value, and determining an approximation of a local maximum value, and should be interpreted as appropriate depending on the context in which the terms are used. Further, the terms include probabilistically or heuristically determining approximations of the maximum values. Similarly, if the terms such as "minimize"/"minimization" are used, they should include determining a global minimum value, determining an approximation of a global minimum value, determining a local minimum value, and determining an approximation of a local minimum value, and should be interpreted as appropriate depending on the context in which the terms are used. Further, the terms include probabilistically or heuristically determining approximations of those minimum values. Similarly, if the terms such as "optimize/optimization" are used, they should include determining a global optimal value, determining an approximation of a global optimal value, determining a local optimal value, and determining an approximation of a local optimal value, and should be interpreted as appropriate depending on the context in which the terms are used. Further, the terms include probabilistically or heuristically determining approximations of those optimal values.

In this specification (including claims), if a plurality of pieces of hardware perform predetermined processing, each piece of hardware may cooperate to perform predetermined processing, or some pieces of hardware may perform all of the predetermined processing. Further, some pieces of hardware may perform part of predetermined processing, and another piece of hardware may perform the rest of the predetermined processing. In this specification (including claims), if the expression such as "one or more pieces of hardware perform first processing and the one or more pieces of hardware perform second processing" (including similar expressions) is used, the hardware that performs the first processing and the hardware that performs the second processing may be the same or different. In other words, the hardware that performs the first processing and the hardware that performs the second processing only need to be included in the one or more pieces of hardware. Note that the hardware may include an electronic circuit, an apparatus including an electronic circuit, or the like.

In this specification (including claims), if a plurality of storage devices (memories) store data, each of the plurality of storage devices may store only part of the data or may store the whole of the data. Further, some storage devices of the plurality of storage devices may include a configuration for storing data.

In this specification (including claims), the terms "first", "second", and the like are used merely as a way of distinguishing between two or more elements, and are not necessarily intended to impose a technical meaning, such as a temporal aspect, a spatial aspect, an order, or a quantity, on the target. Therefore, for example, reference to a first element and a second element does not necessarily mean that only two elements may be employed therein, that the first element should precede the second element, that the first element should be present in order that the second element exists, and the like.

While the embodiment of the present disclosure has been described above in detail, the present disclosure is not limited to the individual embodiment described above. Various additions, modifications, substitutions, partial deletions, and the like may be made without departing from the conceptual spirit and scope of the present invention derived from the contents defined by the scope of claims and their equivalents. For example, in the embodiment described above, if numerical values or mathematical expressions are used in the description, these are shown for the purpose of illustration and do not limit the scope of the present disclosure. Further, the order of the operations described in the embodiment is also exemplary and is not intended to limit the scope of the present disclosure.

The present international application claims priority based on Japanese Patent Application No. 2022-028697 filed on February 25, 2022, and the entire contents of which are incorporated herein by reference.

Reference Signs List

| | |
|---|---|
| 1 | information processing system |
| 10 | information processing apparatus |
| 20 | business operator server |
| 30 | user terminal |
| 31 | user |
| 100 | trained model |
| 110 | training unit |
| 111 | training data acquisition unit |
| 112 | machine leaning unit |
| 120 | estimation unit |
| 121 | body information acquisition unit |
| 122 | missing value estimation unit |
| 123 | estimated value presentation unit |

1001    processor
1002    main storage device (memory)
1003    auxiliary storage device (memory)
1004    network interface
1005    device interface
1010A   external apparatus
1010B   external apparatus

**Claims**

1. An information processing apparatus, comprising:

   at least one memory; and
   at least one processor, wherein
   the at least one processor executes

   acquiring an estimated value of a first attribute by inputting body information in which a value of the first attribute is missing to at least one trained model, and
   acquiring an estimated value of a second attribute by inputting body information in which a value of the second attribute is missing to the at least one trained model, the second attribute being different from the first attribute,

   the at least one trained model is a model that is trained using at least two data sets, and
   the two data sets include missing attributes different from each other and include a value of at least one common attribute.

2. The information processing apparatus according to claim 1, wherein
   the at least two data sets are data sets that are respectively measured in different locations or periods.

3. The information processing apparatus according to claim 1 or 2, wherein
   the first attribute and the second attribute are body information other than basic information.

4. The information processing apparatus according to claim 3, wherein
   the basic information includes sex, age, height, and weight.

5. The information processing apparatus according to any one of claims 1 to 4, wherein
   the body information includes information regarding a physical condition.

6. The information processing apparatus according to any one of claims 1 to 5, wherein
   the body information includes information regarding a psychological state.

7. The information processing apparatus according to any one of claims 1 to 6, wherein
   the estimated value is a value having a predetermined range.

8. The information processing apparatus according to any one of claims 1 to 7, wherein
   the at least one processor acquires a confidence coefficient of the estimated value.

9. The information processing apparatus according to any one of claims 1 to 8, wherein
   the at least one processor provides information regarding the body information on a basis of the estimated value.

10. The information processing apparatus according to any one of claims 1 to 9, wherein
    the at least one processor presents an attribute to be measured to increase the confidence coefficient of the estimated value.

11. The information processing apparatus according to claim 1, wherein
    the at least two data sets are data sets that are measured in different situations.

12. The information processing apparatus according to claim 1, wherein

the at least two data sets are data sets including values of different attributes that are acquired in an identical situation.

13. An information processing apparatus, comprising:

at least one memory; and
at least one processor, wherein
the at least one processor executes

acquiring an estimated value of a first attribute by inputting body information in which a value of the first attribute is missing to at least one trained model,
acquiring an estimated value of a second attribute by inputting body information in which a value of the second attribute is missing to the at least one trained model, the second attribute being different from the first attribute, and
deleting the body information in which the value of the first attribute is missing, the estimated value of the first attribute, the body information in which the value of the second attribute is missing, and the estimated value of the second attribute from the information processing apparatus.

14. The information processing apparatus according to any one of claims 1 to 13, wherein
the at least one processor presents the body information in which the value of the first attribute is missing and the estimated value of the first attribute in a form of table data.

15. The information processing apparatus according to any one of claims 1 to 14, wherein
the at least one processor presents the estimated value of the first attribute in a display form different from a display form of other body information.

16. The information processing apparatus according to any one of claims 1 to 15, wherein
the at least one processor changes the display form of the estimated value of the first attribute on a basis of a confidence coefficient of the estimated value of the first attribute.

17. A terminal, comprising:

at least one memory; and
at least one processor, wherein
the at least one processor executes

transmitting body information in which a value of a first attribute is missing to at least one information processing apparatus,
receiving an estimated value of the first attribute from the at least one information processing apparatus,
transmitting body information in which a value of a second attribute is missing to the at least one information processing apparatus, the second attribute being different from the first attribute, and
receiving an estimated value of the second attribute from the at least one information processing apparatus.

18. The terminal according to claim 17, wherein
the at least one processor further executes

receiving information of an additional attribute from the at least one information processing apparatus,
transmitting a value of the additional attribute to the at least one information processing apparatus, and
receiving a second estimated value from the at least one information processing apparatus, the second estimated value being different from the estimated value of the first attribute, and
the additional attribute is an attribute determined by the at least one information processing apparatus on a basis of the body information in which the value of the first attribute is missing.

19. The terminal according to claim 17 or 18, wherein

the estimated value of the first attribute and the estimated value of the second attribute are calculated using at least one trained model included in the at least one information processing apparatus,
the at least one trained model is a model that is trained using at least two data sets, and
the two data sets include missing attributes different from each other and include a value of at least one common attribute.

**20.** A terminal, comprising:

at least one memory; and
at least one processor, wherein
the at least one processor executes

transmitting body information in which a value of a first attribute is missing to at least one information processing apparatus, and
receiving an estimated value of the first attribute from the at least one information processing apparatus,

the estimated value of the first attribute is calculated using at least one trained model included in the at least one information processing apparatus,
the at least one trained model is a model that is trained using at least two data sets, and
the two data sets include missing attributes different from each other and include a value of at least one common attribute.

**21.** A method that is executed by at least one processor, the method comprising:

acquiring an estimated value of a first attribute by inputting body information in which a value of the first attribute is missing to at least one trained model; and
acquiring an estimated value of a second attribute by inputting body information in which a value of the second attribute is missing to the at least one trained model, the second attribute being different from the first attribute, wherein
the at least one trained model is a model that is trained using at least two data sets, and
the two data sets include missing attributes different from each other and include a value of at least one common attribute.

**22.** A program that causes at least one processor to execute:

acquiring an estimated value of a first attribute by inputting body information in which a value of the first attribute is missing to at least one trained model; and
acquiring an estimated value of a second attribute by inputting body information in which a value of the second attribute is missing to the at least one trained model, the second attribute being different from the first attribute, wherein
the at least one trained model is a model that is trained using at least two data sets, and
the two data sets include missing attributes different from each other and include a value of at least one common attribute.

**23.** A method that is executed by at least one processor, the method comprising:

transmitting body information in which a value of a first attribute is missing to at least one information processing apparatus;
receiving an estimated value of the first attribute from the at least one information processing apparatus;
transmitting body information in which a value of a second attribute is missing to the at least one information processing apparatus, the second attribute being different from the first attribute; and
receiving an estimated value of the second attribute from the at least one information processing apparatus.

**24.** An information processing apparatus, comprising:

at least one memory; and
at least one processor, wherein
the at least one processor executes

acquiring an estimated value of a first attribute by inputting body information in which a value of the first attribute is missing to at least one trained model, and
acquiring an estimated value of a second attribute by inputting body information in which a value of the second attribute is missing to the at least one trained model, the second attribute being different from the first attribute,

the at least one trained model is a model that is trained using at least two data sets,
the two data sets include missing attributes different from each other and include a value of at least one common attribute, and are data sets that are respectively measured in different locations or periods,
the first attribute and the second attribute are body information other than basic information, and
the basic information includes sex, age, height, and weight.

1 Information processing system

31

User

30

User terminal

31

User

30

User terminal

31

User

30

User terminal

20

Business operator
server

10

Information
processing
apparatus

EP 4 485 300 A1

FIG.1

FIG.2

EP 4 485 300 A1

# FIG.3

| | | Missing value | | Overlapping portion |
|---|---|---|---|---|

| ID | Sex | Height | Percentage of body fat | Weight | Blood glucose level | Glucose in urine | Smoking | Age | Stress level | Personality trait | |
|---|---|---|---|---|---|---|---|---|---|---|---|
| 001 | M | 176 | 19.6 | 69.5 | | | | | | | ⎫ |
| 002 | M | 172 | 26.7 | 72.2 | | | | | | | ⎬ Data set 1 |
| 003 | F | 158 | 36.2 | 58.3 | | | | | | | |
| 004 | F | 165 | 24.1 | 54.2 | | | | | | | |
| 005 | F | 152 | 28.4 | 55.4 | | | | | | | ⎭ |
| 006 | | | | 62.3 | 94 | – | No | 42 | | | ⎫ |
| 007 | | | | 59.1 | 132 | + | Yes | 48 | | | |
| 008 | | | | 68.7 | 87 | – | No | 35 | | | ⎬ Data set 2 |
| 009 | | | | 53.2 | 123 | – | No | 56 | | | |
| 010 | | | | 76.8 | 108 | – | Yes | 39 | | | ⎭ |
| 011 | | | | | 90 | | | 26 | 21 | Extroversion | ⎫ |
| 012 | | | | | 112 | | | 47 | 25 | Openness to experience | |
| 013 | | | | | 88 | | | 33 | 28 | Neuroticism | ⎬ Data set 3 |
| 014 | | | | | 130 | | | 49 | 33 | Conscientiousness | |
| 015 | | | | | 118 | | | 52 | 25 | Agreeableness | ⎭ |

# FIG.4

| | Missing value | | Overlapping portion |

| ID | Sex | Height | Receipt information Injuries and diseases (high blood pressure) | Weight | Walking speed | Level of happiness | Scale of sleep | Age | Cognitive function test | Overactive bladder | |
|---|---|---|---|---|---|---|---|---|---|---|---|
| 001 | F | 164 | 3 | 61.2 | | | | | | | ⎫ |
| 002 | F | 151 | 0 | 56.3 | | | | | | | ⎪ |
| 003 | M | 183 | 0 | 72.3 | | | | | | | ⎬ Data set 1 |
| 004 | M | 171 | 6 | 76.8 | | | | | | | ⎪ |
| 005 | M | 175 | 0 | 64.5 | | | | | | | ⎭ |
| 006 | | | | 66.5 | 1.5 | 19 | 3 | 28 | | | ⎫ |
| 007 | | | | 78.2 | 1.4 | 18 | 2 | 33 | | | ⎪ |
| 008 | | | | 57.9 | 1.2 | 12 | 7 | 57 | | | ⎬ Data set 2 |
| 009 | | | | 64.9 | 1.4 | 22 | 1 | 48 | | | ⎪ |
| 010 | | | | 51.8 | 1.3 | 17 | 5 | 64 | | | ⎭ |
| 011 | | | | | 1.4 | | | 38 | 115 | Mild symptom | ⎫ |
| 012 | | | | | 1.2 | | | 66 | 101 | Moderate symptom | ⎪ |
| 013 | | | | | 1.5 | | | 32 | 113 | Mild symptom | ⎬ Data set 3 |
| 014 | | | | | 1.3 | | | 55 | 107 | Mild symptom | ⎪ |
| 015 | | | | | 1.3 | | | 75 | 98 | Mild symptom | ⎭ |

# FIG.5

| | Missing value |
|---|---|

<Body information including missing value>

| ID | Attribute 1 | Attribute 2 | Attribute 3 | Attribute 4 | Attribute 5 | Attribute 6 | Attribute 7 | Attribute 8 | Attribute 9 | Attribute 10 |
|---|---|---|---|---|---|---|---|---|---|---|
| 100 | × × × | × × × | × × × | × × × | | | | | | |
| 200 | | | | × × × | × × × | × × × | × × × | × × × | × × × | × × × |

<Body information including estimated value>

| ID | Attribute 1 | Attribute 2 | Attribute 3 | Attribute 4 | Attribute 5 | Attribute 6 | Attribute 7 | Attribute 8 | Attribute 9 | Attribute 10 |
|---|---|---|---|---|---|---|---|---|---|---|
| 100 | × × × | × × × | × × × | × × × | × × × | × × × | × × × | × × × | × × × | × × × |
| 200 | × × × | × × × | × × × | × × × | × × × | × × × | × × × | × × × | × × × | × × × |

# FIG.6

EP 4 485 300 A1

| | Missing value |
|---|---|

<Body information including missing value>

| ID | Age | Sex | Height | Weight | Area of visceral fat | Neutral fat | Blood glucose level | Stress value | ... | ... |
|---|---|---|---|---|---|---|---|---|---|---|
| 001 | 40 | M | 172 | 68.2 | | | | | ... | ... |
| 002 | 40 | M | 172 | 73.2 | | | | | ... | ... |

| | Estimated value |
|---|---|

<Body information including estimated value>

| ID | Age | Sex | Height | Weight | Area of visceral fat | Neutral fat | Blood glucose level | Stress value | ... | ... |
|---|---|---|---|---|---|---|---|---|---|---|
| 001 | 40 | M | 172 | 68.2 | 78 | 107 | 91 | 32 | ... | ... |
| 002 | 40 | M | 172 | 73.2 | 80 | 116 | 93 | 34 | ... | ... |

```
        ┌─────────────┐
        │    Start    │
        └──────┬──────┘
               │
               ▼                              ⌒S11
    ┌─────────────────────────────────────────────┐
    │                                             │
    │           Acquire training data             │
    │                                             │
    └──────────────────────┬──────────────────────┘
                           │
                           ▼                      ⌒S12
    ┌─────────────────────────────────────────────┐
    │                                             │
    │     Generate trained model by machine learning     │
    │                                             │
    └──────────────────────┬──────────────────────┘
                           │
                           ▼
                    ┌─────────────┐
                    │     End     │
                    └─────────────┘
```

# FIG.7

```
        ┌─────────┐
        │  Start  │
        └─────────┘
             │
             ▼                                        ╭─S21
┌───────────────────────────────────────────────────────┐
│                                                        │
│    Acquire body information including missing value    │
│                                                        │
└───────────────────────────────────────────────────────┘
             │
             ▼                                        ╭─S22
┌───────────────────────────────────────────────────────┐
│                                                        │
│              Estimate missing value                    │
│                                                        │
└───────────────────────────────────────────────────────┘
             │
             ▼                                        ╭─S23
┌───────────────────────────────────────────────────────┐
│                                                        │
│              Present body information                  │
│                                                        │
└───────────────────────────────────────────────────────┘
             │
             ▼
        ┌─────────┐
        │   End   │
        └─────────┘
```

FIG.8

10 Information processing apparatus

FIG.9

EP 4 485 300 A1

## INTERNATIONAL SEARCH REPORT

| International application No. |
|---|
| **PCT/JP2023/006145** |

### A. CLASSIFICATION OF SUBJECT MATTER

*G06Q 10/04*(2023.01)i; *G06Q 50/10*(2012.01)i; *G16H 10/00*(2018.01)i
FI:    G06Q10/04; G06Q50/10; G16H10/00

According to International Patent Classification (IPC) or to both national classification and IPC

### B. FIELDS SEARCHED

Minimum documentation searched (classification system followed by classification symbols)

G06Q10/04; G06Q50/10; G16H10/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2012-248017 A (NIPPON TELEGRAPH AND TELEPHONE CORP.) 13 December 2012 (2012-12-13) entire text, all drawings | 1-24 |
| A | JP 2021-140362 A (OMRON CORP.) 16 September 2021 (2021-09-16) entire text, all drawings | 1-24 |
| A | WO 2019/187933 A1 (NEC SOLUTION INNOVATORS LTD.) 03 October 2019 (2019-10-03) entire text, all drawings | 1-24 |
| A | JP 2021-179865 A (UNIVERISTY OF WASEDA) 18 November 2021 (2021-11-18) entire text, all drawings | 1-24 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 April 2023** | **16 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

| International application No. |
|---|
| **PCT/JP2023/006145** |

| Patent document cited in search report | | | Publication date (day/month/year) | Patent family member(s) | | | Publication date (day/month/year) |
|---|---|---|---|---|---|---|---|
| JP | 2012-248017 | A | 13 December 2012 | (Family: none) | | | |
| JP | 2021-140362 | A | 16 September 2021 | WO entire text, all drawings CN | 2021/176984 115210724 | A1 A | |
| WO | 2019/187933 | A1 | 03 October 2019 | US entire text, all drawings CN | 2021/0257072 111971756 | A1 A | |
| JP | 2021-179865 | A | 18 November 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012064087 A

- JP 2022028697 A

**Non-patent literature cited in the description**

- *Handling Incomplete Heterogeneous Data using VAEs*, https://arxiv.org/pdf/1807.03653.pdf